# EUROPÄISCHE PATENTSCHRIFT

(11) **EP 2 581 112 B1**
(45) Veröffentlichungstag und Bekanntmachung des Hinweises auf die Patenterteilung: **25.05.2016**
(21) Anmeldenummer: 12188106.4
(22) Anmeldetag: 11.10.2012
(51) Int. Cl.: A61N 7/00, G06Q 20/40

(54) **System zum Erzeugen von Ultraschallwellen und Verfahren zur Konfiguration eines Ultraschallsystems**
System for generating ultrasound waves and method for configuring an ultrasound system
Système destiné à générer des ondes à ultrasons et procédé de configuration d'un système à ultrasons

(30) Priorität: 14.10.2011 DE 102011115906
(43) Veröffentlichungstag der Anmeldung: 17.04.2013
(73) Patentinhaber: Wellcomet GmbH, 76229 Karlsruhe (DE)
(72) Erfinder: Kruglikov, Ilja, 76351 Linkenheim-Hochstetten (DE)
(74) Vertreter: Lemcke, Brommer & Partner Patentanwälte Partnerschaft mbB

(56) Entgegenhaltungen:
- EP-A1- 2 441 409
- WO-A1-99/47209
- WO-A1-2010/098868
- US-A1- 2003 232 303
- US-A1- 2005 201 574
- US-A1- 2006 094 988
- US-A1- 2009 248 578
- US-A1- 2009 254 008
- US-A1- 2009 281 464
- US-A1- 2011 112 405

## Beschreibung

Die Erfindung betrifft ein System zum Erzeugen von Ultraschallwellen, insbesondere zur medizinischen und/oder ästhetischen Behandlung.

Ultraschallwellen werden zur Beaufschlagung von biologischem Gewebe für medizinische, therapeutische oder kosmetische Anwendungen sowie für Forschungszwecke benutzt. Üblicherweise werden mit dem Begriff Ultraschallwellen Schwingungen mit einer Frequenz größer 20 kHz bezeichnet. Häufige Anwendung finden hierbei insbesondere sogenannte hochfrequente Ultraschallfrequenzen im Bereich 0,7 MHz bis 20 MHz.

Systeme zur Erzeugung von Ultraschallwellen für die Behandlung von biologischem Gewebe weisen üblicherweise eine Steuereinheit und einen Ultraschallkopf auf. Der Ultraschallkopf ist mittels einer elektrischen Leitung mit einem Signalausgang der Steuereinheit verbunden. Weiterhin besitzt der Ultraschallkopf einen Bereich zur Auflage auf das biologische Gewebe, wobei im Ultraschallkopf in der Nähe dieses Auflagebereiches die von der Steuereinheit erzeugten periodischen elektrischen Signale in Ultraschallwellen umgewandelt werden.

Es sind Systeme zum Erzeugen von Ultraschallwellen bekannt, mittels derer lediglich eine Beaufschlagung des Gewebes mit einer vorgegebenen Ultraschallfrequenz möglich ist.

Aus EP 1 747 818 ist ein System zum Erzeugen von Ultraschallwellen bekannt, welches während der Behandlung einen Wechsel zwischen mehreren Ultraschallfrequenzen mit einer vorgegebenen Wechselfrequenz ermöglicht.

Die vorgenannten Systeme zum Erzeugen von Ultraschallwellen finden bei einer großen Bandbreite von medizinischen Indikationen und/oder kosmetischen Anwendungen Einsatz.

In der US 2003/0232303 wird ein Behandlungsgerät in Form eines Handgerätes gezeigt, welches unter anderem als Ultraschallgerät ausgebildet sein kann. Eine Basisstation ist vorgesehen, um das Handgerät zu laden und eine Datenverbindung mit einem professionellen Dienstleister, herzustellen. Über die Datenverbindung sollen Daten über den Behandlungsverlauf ausgetauscht, aber auch Einstellungen am Behandlungsplan oder Geräteparametern wie beispielsweise der Leistung einer Lichtquelle, vorgenommen werden.

Die US 2011/0112405 zeigt ein Ultraschallbehandlungsgerät, welches ein Handstück mit auswechselbaren Ultraschallköpfen aufweist. Zwischen dem Handstück und einer Steuereinheit kann eine drahtlose Verbindung vorgesehen sein. Ein Betrieb des Ultraschallbehandlungsgerätes ohne die Steuereinheit ist nicht möglich.

Die WO 2010/098868 beschreibt ein implantierbares Behandlungsgerät, welches unter anderem auch zur Abgabe von Ultraschall ausgebildet sein kann, und über eine Funkschnittstelle von einem Computer eines Behandlers konfiguriert werden kann.

In der nicht vorveröffentlichten EP 2 441 409 ist ein als Handgerät ausgebildetes Behandlungsgerät mit einem über ein Spiralkabel verbundenen Ultraschallkopf gezeigt, welches einem Patienten zur Selbstbehandlung mit nach Hause gegeben werden soll. Zur Aktivierung des Gerätes muss der Patient Berechtigungsdaten eingeben, die er vom Behandler erhält. Daneben ist auch die Möglichkeit gegeben, dass die Berechtigungs-Daten über eine Netzwerkverbindung von einem Netzwerkserver erhalten werden.

Der Erfindung liegt daher die Aufgabe zugrunde, die vorbekannten Systeme hinsichtlich des Anwendens für einen Benutzer zu vereinfachen.

Die vorliegende Erfindung und ihre vorteilhaften Ausführungsformen sind im Anspruchssatz definiert.

Die Erfindung ist in der Erkenntnis des Anmelders begründet, dass zwar die große Bandbreite der Anwendung von Ultraschallwellen zur Beaufschlagung von biologischem Gewebe durch die bereits vorbekannten Systeme zum Erzeugen von Ultraschallwellen abgedeckt wird, dass jedoch ein erheblicher Nachteil hinsichtlich der Anwendung an einem Menschen besteht:
Die vorbekannten Geräte sind sehr kostenintensiv und/oder erfordern aufgrund der komplexen Bedienung Fachpersonal, um eine sachgerechte Anwendung der Ultraschallwellen zu gewährleisten und insbesondere um Schäden am biologischen Gewebe durch Verwendung von Ultraschallwellen mit falschen Parametern, insbesondere falsch gewählten Frequenzen, Intensitäten oder Anwendungszeiten zu vermeiden.

Hierdurch ergibt sich eine erhebliche Beeinträchtigung, da der zu Behandelnde für jede Behandlung das Fachpersonal aufsuchen muss. Bei den meisten Anwendungsgebieten bei Beaufschlagung von biologischem Gewebe für medizinische, therapeutische oder kosmetische Anwendung mit Ultraschall ist es jedoch wünschenswert, über einen längeren Zeitraum, beispielsweise zwei oder drei Wochen, eine Vielzahl von Anwendungen, häufig mehrere Anwendungen an einem Tag, durchzuführen. Dies ist bei einer ambulanten Anwendung jedoch kaum praktikabel, da dies eine sehr hohe Einschränkung des zu Behandelnden darstellt, der jeweils das Fachpersonal für die Behandlung aufsuchen muss.

Es besteht daher eine große Notwendigkeit, die vorbekannten Systeme zum Erzeugen von Ultraschallwellen dahingehend zu verbessern, dass diese Nachteile bei einer häufigen ambulanten Behandlung entfallen.

Die Erfindung ist weiterhin in der Erkenntnis des Anmelders begründet, dass insbesondere die Vorgabe der korrekten Behandlungs-Parameter durch das Fachpersonal wesentlich ist und entsprechend insbesondere das Risiko einer Schädigung des zu Behandelnden aufgrund einer Beaufschlagung des biologischen Gewebes mit falsch parametrisiertem Ultraschall erheblich ist.

Das erfindungsgemäße System zum Erzeugen von Ultraschallwellen, insbesondere zur medizinischen und/oder ästhetischen Behandlung umfasst ein Anwendungsgerät mit einer Steuereinheit und einen Ultraschallkopf. Die Steuereinheit ist mit dem Ultraschallkopf derart zusammenwirkend ausgebildet, dass mittels der Steuereinheit Behandlungs-Parameter zum Betrieb des Ultraschallkopfes vorgebbar sind. Hinsichtlich dieses Grundaufbaus entspricht das erfindungsgemäße System somit vorbekannten Systemen.

Wesentlich ist, dass das erfindungsgemäße System eine Programmiereinheit umfasst, welche Programmiereinheit ausgebildet ist zur Programmierung der Steuereinheit mit Behandlungsparametern, das System eine die Programmiereinheit umfassende Basisstation aufweist, welche Basisstation ausgebildet ist, zum Aufbau einer Datenverbindung mit dem Anwendungsgerät, um die Steuereinheit mittels der Programmiereinheit zu programmieren und wobei Anwendungsgerät und Basisstation als separate Einheiten ausgebildet sind.

Außerdem weist die Basisstation einen Einschubschacht für das Anwendungsgerät auf. Insbesondere ist es vorteilhaft, dass Anwendungsgerät und die Basisstation jeweils elektrische Kontakte aufweisen, welche derart angeordnet sind, dass die elektrischen Kontakte bei in dem Einschubschacht der Basisstation angeordnetem Anwendungsgerät zum Datenaustausch elektrisch leitend verbunden sind. Hierdurch ist eine besonders einfache und intuitive Anwendung durch den Arzt oder das geschulte Fachpersonal möglich: Zur Programmierung wird das Anwendungsgerät in den Einschubschacht der Basisstation geführt und hierdurch bereits die Datenverbindung zwischen Programmiereinheit der Basisstation und Steuereinheit des Anwendungsgerätes hergestellt.

Mittels des erfindungsgemäßen Systems zum Erzeugen von Ultraschallwellen ist es somit erstmals möglich, die Ultraschallanwendung durch den zu Behandelnden selbst durchführen zu lassen, wobei das Anwendungsgerät einerseits für eine Mehrzahl unterschiedlicher Anwendungsbereiche durch Vorgabe entsprechender Behandlungs-Parameter programmiert werden kann und andererseits aufgrund der Zugangsbeschränkungseinheit und/oder der separaten Ausbildung von Basisstation und Anwendungsgerät ein Ändern der Behandlungs-Parameter durch den zu Behandelnden ausgeschlossen ist.

Es kann somit durch einen Arzt oder geschultes Fachpersonal mittels der Programmiereinheit die für die jeweilige Behandlung passenden Behandlungs-Parameter in der Steuereinheit programmiert werden und das Anwendungsgerät anschließend dem zu Behandelnden überlassen werden. Insbesondere kann der zu Behandelnde somit beispielsweise mehrmals täglich zu Hause eine Behandlung mittels des Anwendungsgerätes vornehmen, wobei der zu Behandelnde allenfalls mittels eines vorzugsweise an dem Anwendungsgerät angeordneten An- und Ausschalters das Anwendungsgerät in Betrieb nimmt, nicht jedoch eine Änderung und insbesondere nicht eine Fehlvorgabe von Behandlungs-Parametern vornehmen kann. Es ist somit insbesondere keine ambulante Behandlung des zu Behandelnden notwendig. Die persönliche Vorstellung bei dem Arzt oder dem geschulten Fachpersonal kann somit in erheblich größeren Abständen, beispielsweise nach einer Woche, gewählt werden, worauf das Fachpersonal bei Bedarf eine Optimierung der Behandlungs-Parameter mittels der Programmiereinheit vornehmen kann.

Indem das System eine Basisstation aufweist, welche die Programmiereinheit umfasst, wobei Anwendungsgerät und Basisstation als separate Einheiten ausgebildet sind, wird somit allein durch die räumliche Trennung verhindert, dass der zu Behandelnde die Behandlungs-Parameter ändert: Die Basisstation befindet sich bei dem Arzt oder dem geschulten Fachpersonal und dem zu Behandelnden wird lediglich das Anwendungsgerät ausgehändigt, so dass keine Änderung der Behandlungs-Parameter möglich ist.

Außerdem kann die Basisstation eine Programmiereinheit mit Zugangsbeschränkungseinheit aufweisen, so dass zusätzlich eine Bedienung der Programmiereinheit durch unbefugte Personen unterbunden wird. Diese Zugangsbeschränkungseinheit kann in an sich bekannter Weise ausgebildet sein, insbesondere kann der Zugang beispielsweise durch eine Passwortabfrage beschränkt werden. Ebenso liegt es im Rahmen der Erfindung, dass der Zugang mechanisch, beispielsweise mittels einer nur durch einen Schlüssel zu öffnenden Abdeckplatte einer Bedientastatur, beschränkt ist.

Die der Erfindung zugrunde liegende Aufgabe ist weiterhin durch ein Verfahren zur Konfiguration eines Ultraschallsystems, insbesondere zur medizinischen und/oder ästhetischen Behandlung gelöst. Das Verfahren umfasst folgende Verfahrensschritte:
In einem Verfahrensschritt A erfolgt ein Bereitstellen einer Basisstation, umfassend eine Programmiereinheit. In einem Verfahrensschritt B erfolgt ein Vorgeben von mittels der Programmiereinheit zu programmierenden Behandlungs-Parametern. In einem Verfahrensschritt C erfolgt ein Herstellen einer Datenverbindung zwischen einer Steuereinheit eines Anwendungsgerätes und der Programmiereinheit, welches Anwendungsgerät einen mit der Steuereinheit verbundenen Ultraschallkopf aufweist.
In einem Verfahrensschritt D erfolgt ein Programmieren der Steuereinheit mit den Behandlungs-Parametern mittels der Programmiereinheit und in einem Verfahrensschritt E erfolgt ein Lösen der Datenverbindung zwischen der Steuereinheit und der Programmiereinheit.

Mit dem erfindungsgemäßen Verfahren werden somit die vorgenannten Vorteile erzielt, da nach Durchführen des Verfahrensschrittes E, d. h. nach Lösen der Datenverbindung, eine Änderung der Behandlungs-Parameter an dem Anwendungsgerät nicht mehr möglich ist.

Vorzugsweise ist das Steuerungsgerät derart ausgebildet, dass die Behandlungs-Parameter ausschließlich mittels der Programmiereinheit änderbar sind.

Dies kann in einer vorzugsweisen Ausführungsform dadurch realisiert sein, dass in dem Steuerungsgerät und in der Programmiereinheit jeweils eine Kennung hinterlegt ist und Steuereinheit und Programmiereinheit derart ausgebildet sind, dass nur bei Vorliegen einer übereinstimmenden Kennung eine Programmierung erfolgen kann. Hierdurch wird vermieden, dass durch Unbefugte mittels einer weiteren Programmiereinheit eine Änderung von Behandlungs-Parametern erfolgt.

In einer weiteren vorzugsweisen Ausführungsform ist das Anwendungsgerät als Handgerät ausgebildet. Hierdurch ist eine einfache Anwendung von dem zu Behandelten gewährleistet.

Vorzugsweise weist das Anwendungsgerät einen Akku zum Betrieb zumindest der Steuereinheit und des Ultraschallkopfes auf. Hierdurch wird ein erhöhter Komfort bei der Anwendung durch den zu Behandelten erzielt.

Insbesondere bei dieser vorteilhaften Ausführungsform vorteilhaft, wenn zusätzlich ein Ladekabel oder eine Ladestation zum Aufladen des Akkus des Anwendungsgerätes vorgesehen ist.

Die Datenverbindung zwischen Steuereinheit und Programmiereinheit kann in an sich bekannter Weise kabellos erfolgen. Insbesondere ist es vorteilhaft, dass Steuereinheit und Programmiereinheit jeweils eine Funkeinheit zum Aufbau einer Datenverbindung aufweisen.

Vorzugsweise umfasst die Basisstation ein Bedienfeld zur Auswahl von mittels der Programmiereinheit zu programmierenden Behandlungs-Parametern. Hierdurch kann in einfacher Weise eine Vorgabe der Behandlungs-Parameter durch den Arzt oder das geschulte Fachpersonal erfolgen. Ebenso liegt es im Rahmen der Erfindung, dass die Basisstation eine Schnittstelle zur Datenverbindung mit einem Computer aufweist, so dass mittels des Computers die Behandlungs-Parameter vorgegeben werden können.

Ein wesentlicher Aspekt der vorliegenden Erfindung ist - wie vorhergehend beschrieben - dass das Anwendungsgerät polyfunktionell ausgebildet ist, d. h. dass durch Vorgabe unterschiedlicher Behandlungs-Parameter unterschiedliche Einsatzbereiche durch das Anwendungsgerät abgedeckt werden können. Vorzugsweise ist das Anwendungsgerät daher derart ausgebildet, dass die Erzeugung von Ultraschallwellen im Bereich von 0,7 MHz bis 20 MHz vorgebbar ist. Insbesondere ist es vorteilhaft, dass zumindest Ultraschallwellen mit einer Frequenz von etwa 10 MHz erzeugt werden können.

Die mittels der Programmiereinheit vorgebbaren Behandlungs-Parameter umfassen ein oder mehrere der folgenden Parameter:
- eine mittels des Ultraschallkopfes zu erzeugende Ultraschallfrequenz; Vorzugsweise im Bereich 0,7 MHz bis 20 MHz;
- eine Frequenzabfolge von mittels des Ultraschallkopfes zu erzeugende Ultraschallfrequenzen und eine Anwendungszeitdauer für jede Ultraschallfrequenz;
   Vorzugsweise eine Frequenzabfolge von mindestens zwei Frequenzen mit jeweiliger Anwendungsdauer, wobei nach Durchlauf der Frequenzabfolge eine Wiederholung bis zum Erreichen der Gesamtanwendungsdauer erfolgt;
   Vorzugsweise Frequenzabfolgen sind hierbei 5 MHz / 10 MHz;
- vorzugsweise eine Gesamtbehandlungsdauer;
   Vorzugsweise im Bereich 1 Min. bis 30 Min., weiter bevorzugt im Bereich 1 Min. bis 10 Min.;
- eine Intensität des mittels des Ultraschallkopfes zu erzeugenden Ultraschalls;
   Vorzugsweise Intensitäten im Bereich 0,1 W/cm² bis 1 W/cm²;
- Eine Intensitätsabfolge des zu erzeugenden Ultraschalls;
   Vorzugsweise eine Abfolge von mindestens zwei Intensitäten, wobei nach Durchlauf der Intensitätsabfolge eine Wiederholung bis zum Erreichen der Gesamtanwendungsdauer erfolgt;
- Pulslängen zur pulsierenden Erzeugung von Ultraschall mittels des Ultraschallkopfes;
- Maximale Gesamtbehandlungsdauer aller Ultraschallbehandlungen pro Zeiteinheit, vorzugsweise maximale Gesamtbehandlungsdauer aller Ultraschallbehandlungen pro Tag;
- Maximale Anzahl an Ultraschallbehandlungen pro Zeiteinheit, vorzugsweise maximale Anzahl an Ultraschallbehandlungen pro Tag.

Eine Beispiel für eine Behandlungs-Parameter Vorgabe ist: Gesamtbehandlungsdauer 10 Min; Ultraschallfrequenz konstant 10 MHz; Intensität 1 von 0,2 W/cm² für eine Dauer von 5 Minuten bei pulsierendem Betrieb: jeweils 10 ms Ultraschallerzeugung mit einer Pause von 10 ms;
und anschließend Intensität 2 von 0,5 W/cm² für eine Dauer von 5 Minuten bei pulsierendem Betrieb: jeweils 10 ms Ultraschallerzeugung mit einer Pause von 20 ms.

Wie vorhergehend beschrieben ist es vorteilhaft, wenn die Behandlungsparamter auch Parameter hinsichtlich der Durchführung der Ultraschallbehandlung durch den Benutzer umfassen, insbesondere eine maximale Gesamtbehandlungsdauer und/oder eine maximale Anzahl an Ultraschallbehandlungen pro Zeiteinheit. Hierdurch ist es dem Arzt oder Fachpersonal somit möglich, eine zu häufige Anwendung zu unterbinden. Beispielsweise ist es vorteilhaft, das erfindungsgemäße System derart auszubilden, dass eine maximale Anzahl an Behandlungen pro Tag vorgegeben werden kann. Der Benutzer kann an einem Tag somit maximal die vorgegebene Behandlungsanzahl durchführen; ein Versuch, eine weitere, über die maximale Anzahl hinausgehende Behandlung durchzuführen wird verhindert, indem kein Ultraschall bei Einschalten erzeugt wird. Vorzugsweise wird zusätzlich eine optische und/oder akustische Warnmeldung ausgegeben.

Alternativ und/oder zusätzlich ist es vorteilhaft, das erfindungsgemäße System derart auszubilden, dass eine maximale Gesamtbehandlungsdauer pro Zeiteinheit vorgebbar ist. Der Arzt oder das Fachpersonal kann in dieser vorzugsweisen Ausführungsform somit vorgeben, dass pro gewählter Zeiteinheit eine Behandlung maximal für die vorgegebene Gesamtzeitdauer - unabhängig von der Anzahl der Behandlungen - erfolgt. Beispielsweise ist die Vorgabe einer maximalen Gesamtbehandlungszeit pro Tag sinnvoll, so dass der Benutzer an einem Tag unabhängig von der Anzahl der Anwendungen die vorgegebene Gesamtbehandlungsdauer nicht überschreiten kann und das Gewebe somit vor einer zu starken Beanspruchung geschützt ist. Auch hier ist eine Ausbildung des erfindungsgemäßen Systems derart vorteilhaft, dass bei einem Versuch, eine weitere, über die maximale Gesamtdauer hinausgehende Behandlung durchzuführen, verhindert wird, indem kein Ultraschall bei Einschalten erzeugt wird. Vorzugsweise wird zusätzlich eine optische und/oder akustische Warnmeldung ausgegeben.

In einer weiteren vorzugsweisen Ausführungsform umfasst die Steuereinheit eine Speichereinheit, welche ausgebildet ist zur Abspeicherung von Anwendungsdaten, vorzugsweise zur Speicherung einer Anzahl von durchgeführten Ultraschall-Behandlungen. Hierdurch ist es für den Arzt oder das Fachpersonal in einfacher Weise möglich, die Anzahl der durch den Benutzer durchgeführten Behandlungen zu überprüfen.

Insbesondere ist es vorteilhaft, dass die Speichereinheit eine Datumsfunktion und/oder eine Uhrzeitfunktion umfasst und ausgebildet ist zur Speicherung des Datums und/oder der Uhrzeit zu jeder durchgeführten Ultraschall-Behandlung. Auf diese Weise wird jede Anwendung durch den Benutzer automatisch mit Uhrzeit und/oder Datum, vorzugsweise mit Uhrzeit und Datum, protokolliert, so dass der Arzt oder das Fachpersonal eine den Vorgaben entsprechende Behandlung durch den Benutzer überprüfen kann.

Vorzugsweise ist die Speichereinheit ausgebildet zur Datenübertragung der gespeicherten Daten an die Programmiereinheit und die Programmiereinheit ist vorzugsweise ausgebildet zur Anzeige der gespeicherten Daten und/oder zur Weiterleitung der gespeicherten Daten an einen Computer.

Die erfindungsgemäßen Verfahren sind vorzugsweise zu Durchführung mittels des Erfindungsgemäßen Systems bzw. einer vorteilhaften Ausführungsform hiervon ausgebildet. Das Erfindungsgemäße System ist vorzugsweise zur Durchführung eines erfindungsgemäßen Verfahrens bzw. einer vorteilhaften Ausführungsform hiervon ausgebildet.

Weitere Vorteile und vorzugsweise Ausführungsformen der vorliegenden Erfindung werden im Folgenden anhand von Ausführungsbeispielen und den Figuren erläutert. Dabei zeigt:
- Figur 1: ein Anwendungsgerät eines ersten Ausführungsbeispiels eines Systems zum Erzeugen von Ultraschallwellen;
- Figur 2: das Anwendungsgerät aus Figur 1, welches in eine Basisstation des ersten Ausführungsbeispiels eines Systems zum Erzeugen von Ultraschallwellen eingesetzt ist und
- Figur 3: ein zweites Ausführungsbeispiel eines Systems zum Erzeugen von Ultraschallwellen, bei welchem das Anwendungsgerät eine Programmiereinheit zu einer Zugangsbeschränkungseinheit aufweist.

In den Figuren bezeichnen gleiche Bezugselemente gleiche oder gleichwirkende Elemente.

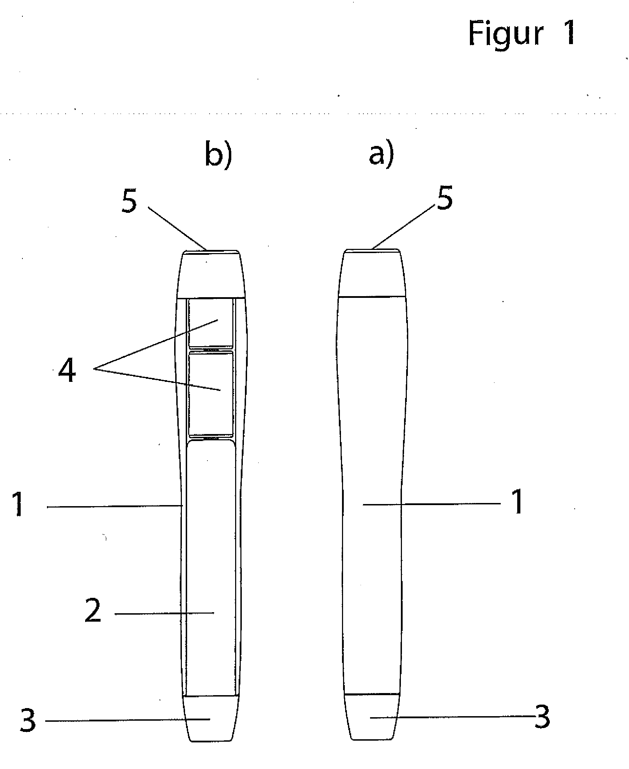

Das erste Ausführungsbeispiel eines Systems zum Erzeugen von Ultraschallwellen weist ein in Figur 1 dargestelltes Anwendungsgerät 1 auf, welches als Handgerät ausgebildet ist. In Figur 1 zeigt Teilbild a eine Darstellung des Anwendungsgerätes 1 und Teilbild b eine Schnittdarstellung des Anwendungsgerätes 1.

Das Anwendungsgerät 1 weist eine Steuereinheit 2 sowie einen Ultraschallkopf 3 auf.

Weiterhin ist ein Aufnahmefach für Akkus 4 vorgesehen, mittels derer Steuereinheit 2 und Ultraschallkopf 3 mit elektrischer Energie versorgt werden.

An der dem Ultraschallkopf 3 gegenüberliegenden Seite ist ein (nicht dargestellter) Ein-/Ausschalter 5 vorgesehen.

Der Benutzer muss somit lediglich an dem Ein-/Ausschalter 5 das Anwendungsgerät 1 einschalten. Anschließend wird mittels der Steuereinheit 2 der Ultraschallkopf 3 derart mit elektrischen Signalen versehen, dass in der Steuereinheit gespeicherte Behandlungs-Parameter in entsprechenden von dem Ultraschallkopf 3 erzeugten Ultraschallwellen realisiert werden. Der Benutzer muss somit lediglich den Ultraschallkopf 3 auf die zu behandelnde Haut bzw. Gewebestellen auflegen, bzw. über diese hinwegführen.

Das Anwendungsgerät 1 gemäß dem in Figur 1 dargestellten Ausführungsbeispiel ist ausgebildet zum Erzeugen von Ultraschallwellen mit einer Frequenz von 10 MHz.

Die vorgebbaren Behandlungsparameter umfassen die Gesamtbehandlungsdauer sowie die Intensität des erzeugten Ultraschalls.

Nach Erreichen der vorgegebenen Behandlungsdauer schaltet sich das Anwendungsgerät 1 über die Steuereinheit 2 automatisch ab.

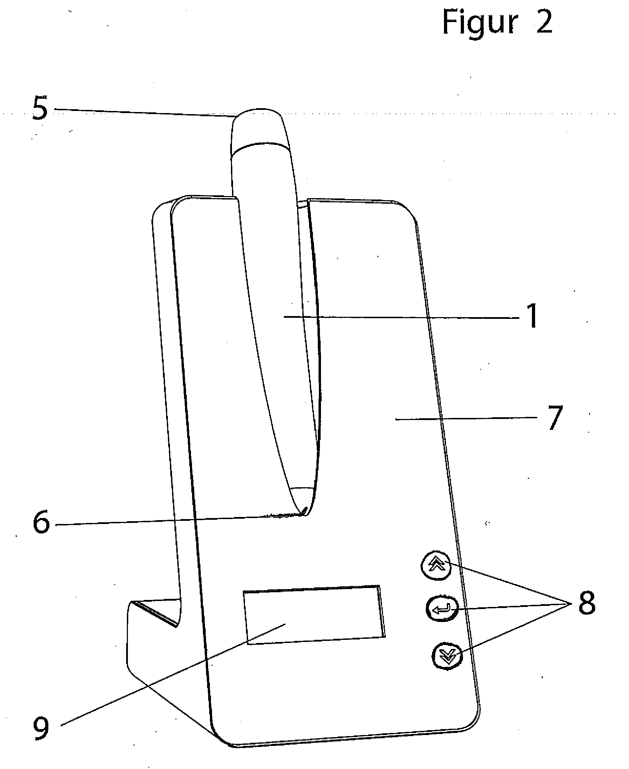

In Figur 2 ist das Behandlungsgerät 1 aus Figur 1 dargestellt, welches in einen Einschubschacht 6 einer Basisstation 7 eingeschoben ist.

Die Basisstation 7 weist Bedienungstasten 8 sowie eine Anzeige 9 auf.

Über - nicht dargestellte - elektrische Kontakte des Anwendungsgerätes 1 und der Basisstation 7 ist eine Datenverbindung zwischen einer - nicht dargestellten - Programmiereinheit der Basisstation 7 und der Steuereinheit 2 des Anwendungsgerätes 1 hergestellt, wenn das Anwendungsgerät 1 in den Einschubschacht 6 der Basisstation 7 eingeschoben ist.

Ein Arzt oder geschultes Fachpersonal kann nun mittels der Bedienungstasten 8 Behandlungsparameter vorgeben, welche über die Programmiereinheit der Basisstation 7 in die Steuereinheit 2 des Anwendungsgerätes 1 programmiert werden.

Anschließend kann das Anwendungsgerät 1 wieder entnommen und dem Benutzer zur weiteren Behandlung mit den neu eingestellten Behandlungs-Parametern ausgehändigt werden.

Zur Kontrolle der vorgebebenen Parameter werden diese bei Auswahl durch die Bedienungstasten 8 auf der Anzeige 9 dargestellt.

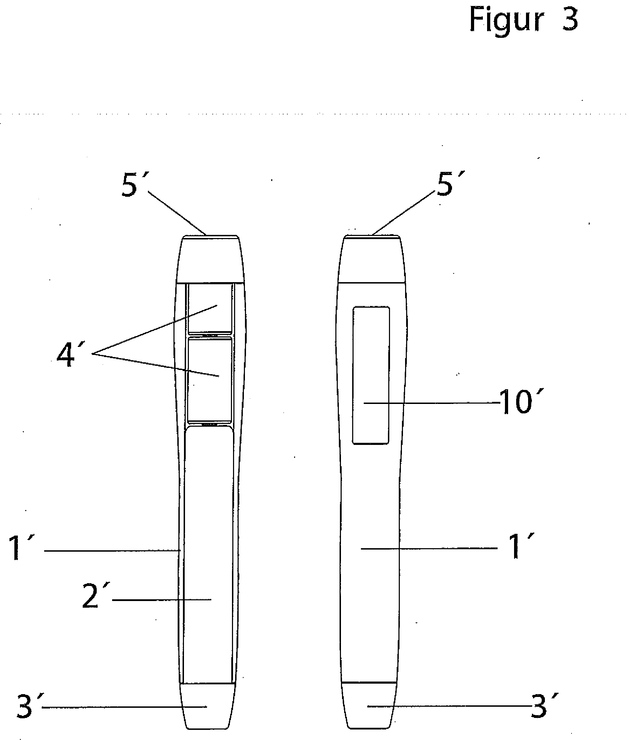

Figur 3 zeigt ein weiteres Ausführungsbeispiel eines erfindungsgemäßen Systems zum Erzeugen von Ultraschallwellen, für welches jedoch im Rahmen der vorliegenden Erfindung nur in Kombination mit dem ersten Ausführungsbeispiel Schutz beansprucht wird.

Bei diesem Ausführungsbeispiel ist ein Anwendungsgerät 1' vorgesehen, welches im Grundaufbau dem Anwendungsgerät 1 gemäß Figur 1 gleicht:
Das Anwendungsgerät 1' weist einen Ultraschallkopf 3', eine Steuereinheit 2' und Akkus 4' auf sowie einen Ein- und Ausschalter 5'.

Im Unterschied zu dem ersten Ausführungsbeispiel ist bei dem zweiten Ausführungsbeispiel auch die Programmiereinheit zusammen mit der Steuereinheit 2' in den Anwendungsgerät 1' integriert. Weiterhin weist das Anwendungsgerät 1' ein berührungssensitives Anzeigenfeld 10' auf, welches somit gleichzeitig zur Dateneingabe und zur Anzeige von Daten verwendet wird.

Bei diesem Ausführungsbeispiel ist somit keine Basisstation notwendig, da die Vorgabe von Behandlungs-Parametern unmittelbar über das berührungssensitive Anzeigenfeld (Touch-Display) 10' erfolgt.

Um eine Änderung der Behandlungs-Parameter durch unbefugte Personen und insbesondere durch den Benutzer zu verhindern, weist die Programmiereinheit des Anwendungsgerätes 1' eine Zugangsbeschränkungseinheit auf. Diese ist derart ausgebildet, dass zunächst eine Passworteingabe erfolgen muss und erst nach Eingabe des korrekten Passworts eine Änderung der Behandlungs-Parameter möglich ist.

Bei diesem Ausführungsbeispiel wird der Arzt oder das geschulte Fachpersonal somit unmittelbar über das Anzeigenfeld 10' nach Entsperren der Zugangsbeschränkung durch Eingabe des Passworts die Behandlungsparameter vorgeben und anschließend wieder die Zugangsbeschränkung aktivieren.

Der Benutzer, welchem das Passwort nicht bekannt ist, kann lediglich über Ein- und Ausschalten des Anwendungsgerätes 1' mittels des Ein-/Ausschalters 5', wie bei dem ersten Ausführungsbeispiel beschrieben, eine Erzeugung von Ultraschall mit den vorgegebenen Behandlungs-Parametern starten.

## Patentansprüche

1. System zum Erzeugen von Ultraschallwellen zur medizinischen und/oder ästhetischen Behandlung,
umfassend ein Anwendungsgerät (1, 1') mit einer Steuereinheit (2, 2') und einem Ultraschallkopf (3, 3'), wobei die Steuereinheit (2, 2') mit dem Ultraschallkopf (3, 3') derart zusammenwirkend ausgebildet ist, dass mittels der Steuereinheit (2, 2') Behandlungs-Parameter zum Betrieb des Ultraschallkopfes vorgebbar sind, wobei das System eine Programmiereinheit umfasst, die zur Programmierung der Steuereinheit (2, 2') mit Behandlungs-Parametern ausgebildet ist, wobei das System eine die Programmiereinheit umfassende Basisstation (7) aufweist, wobei das Anwendungsgerät (1, 1') und die Basisstation (7) als separate Einheiten ausgebildet sind, und wobei die Basisstation (7) einen Einschubschacht (6) für das Anwendungsgerät (1, 1') aufweist und zum Aufbau einer Datenverbindung mit dem Anwendungsgerät (1, 1') ausgebildet ist, wenn das Anwendungsgerät (1, 1') in den Einschubschacht (6) der Basisstation (7) eingeschoben ist, um die Steuereinheit (2, 2') mittels der Programmiereinheit zu programmieren, und
wobei mittels der Programmiereinheit ein oder mehrere der folgenden Ultraschall-Behandlungs-Parameter für unterschiedliche Einsatzbereiche vorgebbar sind:
- eine mittels des Ultraschallkopfes zu erzeugende Ultraschallfrequenz, auswählbar aus einem Frequenzbereich je nach Einsatzbereich;
- eine Frequenzabfolge von mittels des Ultraschallkopfes zu erzeugende Ultraschallfrequenzen und eine Anwendungszeitdauer für jede Ultraschallfrequenz;
- eine Intensität oder eine Intensitätsabfolge des mittels des Ultraschallkopfes zu erzeugenden Ultraschalls, je nach Einsatzbereich;
- Pulslängen zur pulsierenden Erzeugung von Ultraschall mittels des Ultraschallkopfes;
- Maximale Gesamtbehandlungsdauer aller Ultraschallbehandlungen pro Zeiteinheit;
- Maximale Anzahl an Ultraschallbehandlungen pro Zeiteinheit.

2. System nach Anspruch 1,
**dadurch gekennzeichnet,**
**dass** die Steuereinheit (2, 2') derart ausgebildet ist, dass die Behandlungs-Parameter ausschließlich mittels der Programmiereinheit änderbar sind.

3. System nach einem der vorangegangenen Ansprüche,
**dadurch gekennzeichnet,**
**dass** das Anwendungsgerät (1, 1') als Handgerät ausgebildet ist.

4. System nach einem der vorangegangenen Ansprüche,
**dadurch gekennzeichnet,**
**dass** das Anwendungsgerät (1, 1') einen Akku zum Betrieb zumindest der Steuereinheit (2, 2') und des Ultraschallkopfes aufweist.

5. System nach einem der vorangegangenen Ansprüche,
**dadurch gekennzeichnet,**
**dass** Steuereinheit (2, 2') und Programmiereinheit jeweils eine Funkeinheit zum Aufbau einer Datenverbindung aufweisen.

6. System nach einem der vorangegangenen Ansprüche,
**dadurch gekennzeichnet,**
**dass** das Anwendungsgerät (1, 1') und die Basisstation (7) jeweils elektrische Kontakte aufweisen, welche derart angeordnet sind, dass die elektrischen Kontakte bei in dem Einschubschacht (6) der Basisstation (7) angeordnetem Anwendungsgerät (1, 1') zum Datenaustausch elektrisch leitend verbunden sind.

7. System nach einem der vorangegangenen Ansprüche,
**dadurch gekennzeichnet,**
**dass** die Basisstation (7) ein Bedienfeld zur Auswahl von mittels der Programmiereinheit zu programmierenden Behandlungs-Parametern aufweist.

8. System nach einem der vorangegangenen Ansprüche,
**dadurch gekennzeichnet,**
**dass** das Anwendungsgerät (1, 1') zur Erzeugung von Ultraschallwellen im Bereich 0,7 MHz bis 20 MHz ausgebildet ist.

9. System nach Anspruch 8,
**dadurch gekennzeichnet,**
**dass** das Anwendungsgerät (1, 1') zur Erzeugung von Ultraschallwellen im Bereich von etwa 10 MHz ausgebildet ist.

10. System nach einem der vorangegangenen Ansprüche,
**dadurch gekennzeichnet,**
**dass** die Behandlungs-Parameter eine Gesamtbehandlungsdauer umfassen.

11. System nach einem der vorangegangenen Ansprüche,
**dadurch gekennzeichnet,**
**dass** die Steuereinheit eine Speichereinheit umfasst, welche ausgebildet ist zur Abspeicherung von Anwendungsdaten.

12. System nach Anspruch 11,
**dadurch gekennzeichnet,**
**dass** die Speichereinheit zur Speicherung einer Anzahl von durchgeführten Ultraschall-Behandlungen ausgebildet ist.

13. System nach Anspruch 12,
**dadurch gekennzeichnet,**
**dass** die Speichereinheit eine Datumsfunktion und/oder eine Uhrzeitfunktion umfasst und ausgebildet ist zur Speicherung des Datums und/oder der Uhrzeit zu jeder durchgeführten Ultraschall-Behandlung.

14. System nach einem der vorangegangenen Ansprüche,
**dadurch gekennzeichnet,**
**dass** die Programmiereinheit eine Zugangsbeschränkungseinheit aufweist.

15. Verfahren zur Konfiguration eines Ultraschallsystems zur medizinischen und/oder ästhetischen Behandlung, folgende Verfahrensschritte umfassend:
A Bereitstellen einer Basisstation (7), umfassend eine Programmiereinheit und einen Einschubschacht (6) für ein Anwendungsgerät (1, 1 ),
B Vorgeben von mittels der Programmiereinheit zur programmierenden Behandlungs-Parametern;
C Herstellen einer Datenverbindung zwischen einer Steuereinheit (2, 2') des Anwendungsgerätes (1, 1') und der Programmiereinheit, durch Einschieben des Anwendungsgeräts (1, 1') in den Einschubschacht (6) der Basisstation (7), wobei das Anwendungsgerät (1, 1') einen mit der Steuereinheit (2, 2') verbundenen Ultraschallkopf (3, 3') aufweist;
D Programmieren der Steuereinheit (2, 2') mit den Behandlungs-Parametern mittels der Programmiereinheit und
E Lösen der Datenverbindung zwischen der Steuereinheit (2, 2') und der Programmiereinheit durch Entnehmen des Anwendungsgerät (1, 1') aus dem Einschubschacht (6), wobei mittels des Programmiereinheit ein oder mehrere der folgenden Ultraschall-Behandlungs-Parameter abhängig von einem Einsatzbereich vorgegeben wird:
- Eine mittels des Ultraschallkopfes zu erzeugende Ultraschallfrequenz, ausgewählt aus einem Frequenzbereich je nach Einsatzbereich;
- Eine Frequenzabfolge von mittels des Ultraschallkopfes zu erzeugende Ultraschallfrequenzen und eine Anwendungszeitdauer für jede Ultraschallfrequenz;
- Eine Intensität oder eine Intensitätsabfolge des mittels des Ultraschallkopfes zu erzeugenden Ultraschalls, je nach Einsatzbereich;
- Pulslängen zur pulsierenden Erzeugung von Ultraschall mittels des Ultraschallkopfes;
- Eine maximale Gesamtbehandlungsdauer aller Ultraschallbehandlungen pro Zeiteinheit;
- Eine maximale Anzahl an Ultraschallbehandlungen pro Zeiteinheit.

## Claims

1. System for generating ultrasonic waves for medical and/or aesthetic treatment,
comprising an application device (1, 1') having a control unit (2, 2') and an ultrasound head (3, 3'), the control unit (2, 2') being configured to co-operate with the ultrasound head (3, 3') in such a way that by means of the control unit (2, 2') it is possible to specify treatment parameters for operation of the ultrasound head,
wherein the system comprises a programming unit which is configured for programming the control unit (2, 2') with treatment parameters, the system having a base station (7) comprising the programming unit, wherein the application device (1, 1') and the base station (7) are in the form of separate units, and wherein the base station (7) has an insertion slot (6) for the application device (1, 1') and is configured for establishing a data connection with the application device (1, 1') when the application device (1, 1') has been inserted into the insertion slot (6) of the base station (7) in order to program the control unit (2, 2') by means of the programming unit, and
wherein by means of the programming unit it is possible to specify one or more of the following ultrasound treatment parameters for different fields of use:
- an ultrasound frequency to be generated by means of the ultrasound head, selectable from a frequency range depending upon the field of use;
- a frequency sequence of ultrasound frequencies to be generated by means of the ultrasound head and an application time period for each ultrasound frequency;
- an intensity or an intensity sequence of the ultrasound to be generated by means of the ultrasound head, depending upon the field of use;
- pulse lengths for pulsed generation of ultrasound by means of the ultrasound head;
- maximum total treatment period of all ultrasound treatments per unit time;
- maximum number of ultrasound treatments per unit time.

2. System according to claim 1,
**characterised in that**
the control unit (2, 2') is configured in such a way that the treatment parameters can be varied exclusively by means of the programming unit.

3. System according to either one of the preceding claims,
**characterised in that**
the application device (1, 1') is in the form of a hand-held device.

4. System according to any one of the preceding claims,
**characterised in that**
the application device (1, 1') has a battery for operation of at least the control unit (2, 2') and the ultrasound head.

5. System according to any one of the preceding claims,
**characterised in that**
the control unit (2, 2') and the programming unit each have a radio unit for establishing a data connection.

6. System according to any one of the preceding claims,
**characterised in that**
the application device (1, 1') and the base station (7) each have electrical contacts which are arranged in such a way that when the application device (1, 1') is arranged in the insertion slot (6) of the base station (7) the electrical contacts are in electrically conductive connection for data exchange.

7. System according to any one of the preceding claims,
**characterised in that**
the base station (7) has an operating panel for selection of treatment parameters to be programmed by means of the programming unit.

8. System according to any one of the preceding claims,
**characterised in that**
the application device (1, 1') is configured to generate ultrasonic waves in the range of from 0.7 MHz to 20 MHz.

9. System according to claim 8,
**characterised in that**
the application device (1, 1') is configured to generate ultrasonic waves in the region of about 10 MHz.

10. System according to any one of the preceding claims,
**characterised in that**
the treatment parameters comprise a total treatment period.

11. System according to any one of the preceding claims,
**characterised in that**
the control unit comprises a memory unit which is configured to store application data.

12. System according to claim 11,
**characterised in that**
the memory unit is configured to store a number of ultrasound treatments performed.

13. System according to claim 12,
**characterised in that**
the memory unit comprises a date function and/or a time function and is configured to store the date and/or the time for each ultrasound treatment performed.

14. System according to any one of the preceding claims,
**characterised in that**
the programming unit has an access restriction unit.

15. Method of configuring an ultrasound system for medical and/or aesthetic treatment,
comprising the following method steps:
A providing a base station (7), comprising a programming unit and an insertion slot (6) for an application device (1, 1');
B specifying treatment parameters to be programmed by means of the programming unit;
C establishing a data connection between a control unit (2, 2') of the application device (1, 1') and the programming unit, by inserting the application device (1, 1') into the insertion slot (6) of the base station (7), the application device (1, 1') having an ultrasound head (3, 3') connected to the control unit (2, 2');
D programming the control unit (2, 2') with the treatment parameters by means of the programming unit; and
E breaking the data connection between the control unit (2, 2') and the programming unit by removing the application device (1, 1') from the insertion slot (6), one or more of the following ultrasound treatment parameters being specified by means of the programming unit depending upon a field of use:
- an ultrasound frequency to be generated by means of the ultrasound head, selected from a frequency range depending upon the field of use;
- a frequency sequence of ultrasound frequencies to be generated by means of the ultrasound head and an application time period for each ultrasound frequency;
- an intensity or an intensity sequence of the ultrasound to be generated by means of the ultrasound head, depending upon the field of use;
- pulse lengths for pulsed generation of ultrasound by means of the ultrasound head;
- a maximum total treatment period of all ultrasound treatments per unit time;
- a maximum number of ultrasound treatments per unit time.

## Revendications

1. Système destiné à générer des ondes ultrasonores pour un traitement médical et/ou esthétique,
comprenant un appareil d'application (1, 1') avec une unité de commande (2, 2') et une tête à ultrasons (3, 3'), l'unité de commande (2, 2') étant conçue pour coopérer avec la tête à ultrasons (3, 3') de façon que des paramètres de traitement pour le fonctionnement de la tête à ultrasons puissent être prescrits au moyen de l'unité de commande (2, 2'),
le système comprenant une unité de programmation qui est conçue pour programmer l'unité de commande (2, 2') avec des paramètres de traitement, le système présentant une station de base (7) comprenant l'unité de programmation, l'appareil d'application (1, 1') et la station de base (7) étant conçus comme des unités séparées, et la station de base (7) présentant un compartiment (6) pour l'appareil d'application (1, 1') et étant conçue pour établir une liaison de données avec l'appareil d'application (1, 1') lorsque l'appareil d'application (1, 1') est inséré dans le compartiment (6) de la station de base (7), afin de programmer l'unité de commande (2, 2') au moyen de l'unité de programmation, et
un ou plusieurs des paramètres de traitement par ultrasons suivants pour différents domaines d'utilisation pouvant être prescrits au moyen de l'unité de programmation :
- une fréquence d'ultrasons à produire au moyen de la tête à ultrasons, sélectionnable dans une gamme de fréquences en fonction du domaine d'utilisation ;
- une séquence de fréquences d'ultrasons à produire au moyen de la tête à ultrasons et une durée d'application pour chaque fréquence d'ultrasons ;
- une intensité ou une séquence d'intensités des ultrasons à produire au moyen de la tête à ultrasons, en fonction du domaine d'utilisation ;
- des longueurs d'impulsion pour la production pulsée d'ultrasons au moyen de la tête à ultrasons ;
- une durée maximale de traitement de tous les traitements par ultrasons par unité de temps ;
- un nombre maximal de traitements par ultrasons par unité de temps.

2. Système selon la revendication 1,
**caractérisé en ce**
**que** l'unité de commande (2, 2') est conçue de façon que les paramètres de traitement soient modifiables exclusivement au moyen de l'unité de programmation.

3. Système selon l'une des revendications précédentes,
**caractérisé en ce**
**que** l'appareil d'application (1, 1') est conçu comme un appareil portatif.

4. Système selon l'une des revendications précédentes,
**caractérisé en ce**
**que** l'appareil d'application (1, 1') présente un accumulateur pour le fonctionnement au moins de l'unité de commande (2, 2') et de la tête à ultrasons.

5. Système selon l'une des revendications précédentes,
**caractérisé en ce**
**que** l'unité de commande (2, 2') et l'unité de programmation présentent chacune une unité radio pour établir une liaison de données.

6. Système selon l'une des revendications précédentes,
**caractérisé en ce**
**que** l'appareil d'application (1, 1') et la station de base (7) présentent chacun des contacts électriques qui sont disposés de façon que les contacts électriques soient reliés de manière électriquement conductrice pour l'échange de données lorsque l'appareil d'application (1, 1') est disposé dans le compartiment (6) de la station de base (7).

7. Système selon l'une des revendications précédentes,
**caractérisé en ce**
**que** la station de base (7) présente un panneau de commande pour la sélection de paramètres de traitement à programmer au moyen de l'unité de programmation.

8. Système selon l'une des revendications précédentes,
**caractérisé en ce**
**que** l'appareil d'application (1, 1') est conçu pour produire des ondes ultrasonores dans la gamme de 0,7 MHz à 20 MHz.

9. Système selon la revendication 8,
**caractérisé en ce**
**que** l'appareil d'application (1, 1') est conçu pour produire des ondes ultrasonores au voisinage d'environ 10 MHz.

10. Système selon l'une des revendications précédentes,
**caractérisé en ce**
**que** les paramètres de traitement comprennent une durée totale de traitement.

11. Système selon l'une des revendications précédentes,
**caractérisé en ce**
**que** l'unité de commande comprend une unité de mémoire qui est conçue pour mémoriser des données d'application.

12. Système selon la revendication 11,
**caractérisé en ce**
**que** l'unité de mémoire est conçue pour mémoriser un nombre de traitements par ultrasons effectués.

13. Système selon la revendication 12,
**caractérisé en ce**
**que** l'unité de mémoire comprend une fonction de date et/ou une fonction d'heure et est conçue pour mémoriser la date et/ou l'heure de chaque traitement par ultrasons effectué.

14. Système selon l'une des revendications précédentes,
**caractérisé en ce**
**que** l'unité de programmation présente une unité de restriction d'accès.

15. Procédé de configuration d'un système à ultrasons pour un traitement médical et/ou esthétique,
lequel procédé comprend les étapes suivantes :
A mise à disposition d'une station de base (7) comprenant une unité de programmation et un compartiment (6) pour un appareil d'application (1, 1') ;
B prescription de paramètres de traitement à programmer au moyen de l'unité de programmation ;
C établissement d'une liaison de données entre une unité de commande (2, 2') de l'appareil d'application (1, 1') et l'unité de programmation par insertion de l'appareil d'application (1, 1') dans le compartiment (6) de la station de base (7), l'appareil d'application (1, 1') présentant une tête à ultrasons (3, 3') reliée à l'unité de commande (2, 2') ;
D programmation de l'unité de commande (2, 2') avec les paramètres de traitement au moyen de l'unité de programmation et
E libération de la liaison de données entre l'unité de commande (2, 2') et l'unité de programmation par enlèvement de l'appareil d'application (1, 1') du compartiment (6), un ou plusieurs des paramètres de traitement par ultrasons suivants étant prescrits en fonction d'un domaine d'utilisation au moyen de l'unité de programmation :
- une fréquence d'ultrasons à produire au moyen de la tête à ultrasons, sélectionnée dans une gamme de fréquences en fonction du domaine d'utilisation ;
- une séquence de fréquences d'ultrasons à produire au moyen de la tête à ultrasons et une durée d'application pour chaque fréquence d'ultrasons ;
- une intensité ou une séquence d'intensités des ultrasons à produire au moyen de la tête à ultrasons, en fonction du domaine d'utilisation ;
- des longueurs d'impulsion pour la production pulsée d'ultrasons au moyen de la tête à ultrasons ;
- une durée maximale de traitement de tous les traitements par ultrasons par unité de temps ;
- un nombre maximal de traitements par ultrasons par unité de temps.
